# EUROPÄISCHE PATENTSCHRIFT

(11) **EP 1 638 532 B1**
(45) Veröffentlichungstag und Bekanntmachung des Hinweises auf die Patenterteilung: **25.02.2009**
(21) Anmeldenummer: 04739918.3
(22) Anmeldetag: 16.06.2004
(51) Int. Cl.: A61K 9/19, A61K 31/44

(54) **INJIZIERBARE DARREICHUNGSFORM VON FLUPIRTIN**
FLUPIRTIN INJECTABLE GALENIC FORM
FORME GALENIQUE INJECTABLE DE FLUPIRTINE

(30) Priorität: 20.06.2003 DE 10327674
(43) Veröffentlichungstag der Anmeldung: 29.03.2006
(60) Teilanmeldung: 08010996.0
(73) Patentinhaber: AWD.pharma GmbH & Co.KG, 01445 Radebeul (DE)
(72) Erfinder: PIEROTH, Michael, 01689 Weinböhla (DE); STANG, Norbert, 01737 Oberhermsdorf (DE); THOMA, Rudy, 14823 Lühnsdorf (DE); BLUME, Henning, 61325 Bad Homburg (DE)
(74) Vertreter: Bublak, Wolfgang
(86) Internationale Anmeldenummer: PCT/EP2004/006449
(87) Internationale Veröffentlichungsnummer: WO 2004/112754

(56) Entgegenhaltungen:
- WO-A-02/080912
- DE-A- 3 416 609

## Beschreibung

Die vorliegende Erfindung betrifft ein Flupirtin-haltiges Lyophilisat, die Verwendung des Lyophilisats zur Herstellung einer parenteral zu verabreichenden pharmazeutischen Zusammensetzung, ein Verfahren zur Herstellung einer parenteral zu verabreichenden Flupirtin-haltigen pharmazeutischen Zusammensetzung, ein Verfahren zur Herstellung des Flupirtin-haltigen Lyophilisates sowie die unter Verwendung des Lyophilisates hergestellte Flupirtin-haltige pharmazeutische Zusammensetzung.

Flupirtin (Katadolon^{®}; 2-Amino-3-carbethoxyamino-6-(4-fluorbenzylamino)-pyridin = 2-Amino-3-ethoxycarbonylamino-6-(p-fluorbenzylamino)-pyridin) ist ein zentral wirksames, nicht-opiates Analgetikum. Flupirtin, insbesondere in Form seines Maleinsäure-Salzes, wird seit vielen Jahren erfolgreich zur Therapie von beispielsweise Nervenschmerzen, Schmerzen bei abnutzungsbedingten Gelenkerkrankungen, Kopfschmerzen und postoperativen Schmerzen eingesetzt. Gemäß DE 41 22 166 A1 kann Flupirtin auch als Mittel zur Bekämpfung von Erkrankungen oder Krankheitssymptomen, die auf Muskelverspannungen beruhen oder eine Folge solcher Muskelverspannungen sind, eingesetzt werden. In der DE 43 27 516 ist des weiteren die Verwendung von Flupirtin zur Behandlung von cerebraler Ischämie und neurodegenerativen Erkrankungen beschrieben. Aus der DE 195 41 405 A1 ist die Verwendung von Flupirtin zur Prophylaxe und Therapie von Erkrankungen, die mit einer Beeinträchtigung des hämatopoetischen Zellsystems einhergehen, bekannt. Die DE 100 48 969 A1 beschreibt ferner die Verwendung von Flupirtin zur Tinnitus-Behandlung. Die Herstellung von Flupirtin und physiologisch verwendbarer Salze davon ist in DE 17 95 858 C2, DE 31 33 519 C2 und DE 34 16 609 A1 beschrieben.

Flupirtin wird hauptsächlich oral appliziert. So beschreibt die DE 93 21 574 U1 beispielsweise Arzneimittel-Formulierungen in Form von Tabletten, Granulaten oder Pellets, die Flupirtinmaleat als Wirkstoff enthalten. Aus der DE 43 19 649 A1 sind feste Flupirtin-haltige orale Darreichungsformen mit kontrollierter Wirkstoffabgabe bekannt.

Aufgrund der guten schmerzstillenden Wirkung von Flupirtin ist es jedoch erstrebenswert, Flupirtin parenteral zu verabreichen, um eine schnelle lokale oder systemische Wirkung zu erzielen. Dem steht jedoch entgegen, dass Flupirtin beziehungsweise physiologisch wirksame Salze davon in wässrigen Lösungen und in den meisten physiologisch verträglichen organischen Lösungsmitteln kaum löslich sind.

Die DE 34 16 609 A1 beschreibt pharmazeutische Formulierungen in Form injizierbarer Flupirtin-Gluconat-Lösungen, die unter Verwendung geeigneter Lösungsmittel hergestellt werden. Als Lösungsmittel wird insbesondere ein Gemisch aus Polyethylenglykol und Wasser oder ein Gemisch aus Glycofurol und Wasser eingesetzt. Die beschriebenen Injektionslösungen weisen jedoch eine Reihe gravierender Nachteile auf. So sind die unter Verwendung der Polyethylenglykol/Wasser- oder Glycofurol/Wasser-Gemische hergestellten Flupirtin-Gluconat-Lösungen extrem hypertonisch und daher nur zur intramuskulären Anwendung geeignet. Bedingt durch den relativ niedrigen pH-Wert von 3,2 bis 3,6 und die eingesetzten Hilfsstoffe wie Natriumdisulfit und Propylenglykol kommt es auch häufig zu Irritationen an der Applikationsstelle. Darüber hinaus weisen die beschriebenen Flupirtin-Gluconat-Lösungen eine nicht ausreichende physikalische Stabilität auf, da sie nur über einen sehr begrenzten Zeitraum stabil sind und bereits nach wenigen Wochen ein Präzipitationsprozess einsetzt, der die Haltbarkeit des Fertigpräparates deutlich limitiert. Darüber hinaus hat sich herausgestellt, dass die physikalische Stabilität der Flupirtin-Lösungen in hohem Maße auch von der Lagertemperatur abhängig ist. Da die Präzipitation bei tieferen Temperaturen deutlich früher einsetzt, ist es erforderlich, bei der Lagerung der Flupirtin-Lösungen eine Mindesttemperatur von 20°C einzuhalten, um die Lagerungsstabilität zu verbessern. Idealerweise müssten solche Injektionslösungen enthaltenden Ampullen bei Temperaturen von 25°C bis 30°C gelagert werden, was in der Praxis jedoch kaum zu realisieren ist.

Das der vorliegenden Anmeldung zugrunde liegende technische Problem besteht daher darin, Mittel und Verfahren zur Herstellung Flupirtin-haltiger pharmazeutischer Zusammensetzungen bereitzustellen, die zur parenteralen Verabreichung geeignet sind und die nicht die im Stand der Technik bekannten Nachteile parenteraler pharmazeutischer Zusammensetzungen aufweisen, das heißt, insbesondere bei Applikation keine Nebenwirkungen wie Irritationen hervorrufen und darüber hinaus über einen ausreichend langen Zeitraum physikalisch und chemisch stabil sind.

Die vorliegende Erfindung löst das ihr zugrunde liegende technische Problem durch die Bereitstellung eines Lyophilisates, das den Wirkstoff Flupirtin in Basen-Form oder als physiologisch verträgliches Salz enthält und das zur Herstellung einer parenteral zu verabreichenden pharmazeutischen Zusammensetzung eingesetzt werden kann.

Erfindungsgemäß wurde überraschenderweise gefunden, dass die im Stand der Technik bekannten Nachteile und Probleme bei der Handhabung und Lagerung von Flupirtin-Injektionslösungen durch Verwendung des erfindungsgemäßen Flupirtin-Lyophilisates zur Herstellung parenteral zu verabreichender, flüssiger Formulierungen vollständig behoben werden können. So führt die Rekonstitution des erfindungsgemäßen Flupirtin-Lyophilisates in vorteilhafter Weise zu sehr klaren Flupirtin-Lösungen, die über mehrere Stunden hinweg stabil sind und keine Präzipitationsprozesse zeigen, während bei konventionellen Injektionslösungen Flupirtin schon unmittelbar nach Lösungseintritt präzipitiert. Aufgrund ihrer Stabilität sind die unter Verwendung der erfindungsgemäßen Flupirtin-Lyophilisate hergestellten Flupirtin-Lösungen daher in hervorragender Weise zur parenteraler Verabreichung, insbesondere als Injektions- oder Infusionslösungen einsetzbar.

Ein weiterer überraschender Vorteil der erfindungsgemäßen Flupirtin-Lyophilisate besteht darin, dass zur Herstellung flüssiger Darreichungsformen rein wässrige Medien eingesetzt werden können. Während bei der Herstellung herkömmlicher Flupirtin-Injektionslösungen keine rein wässrigen Medien verwendet werden können, sondern nur Lösungsmittelsysteme, die einen hohen Anteil an organischen Lösungsmitteln wie Propylenglykol aufweisen, sind die erfindungsgemäßen Flupirtin-Lyophilisate in wässrigen Systemen hervorragend löslich, so dass zum Lösen keine organischen Lösungsmittel oder lösungsvermittelnden Substanzen eingesetzt werden müssen. Die erfindungsgemäßen Flupirtin-Lyophilisate weisen zudem den Vorteil auf, dass ein Erwärmen beim Lösen des Lyophilisates nicht erforderlich ist, da die erfindungsgemäßen Flupirtin-Lyophilisate bereits bei Raumtemperatur sehr schnell in Lösung gehen.

Ein weiterer Vorteil besteht darin, dass die erfindungsgemäßen Lyophilisate nach Belieben rekonstituiert und/oder verdünnt werden können. So kann das erfindungsgemäße Flupirtin-Lyophilisat gleichermaßen zur Herstellung von intramuskulären oder intravenösen Injektionslösungen, aber auch zur Zubereitung von Infusionslösungen eingesetzt werden. Dabei kann die rekonstituierte wässrige Zubereitung auch als Zumischung zu üblicherweise verwendeten Infusionslösungen verwendet werden. Diese Form der Anwendung ist vor allem für solche Patienten von Vorteil, für die eine systemische Schmerzbehandlung im Zusammenhang mit anderen therapeutischen Maßnahmen erforderlich ist. Da sich die erfindungsgemäßen Lyophilisate in charakteristischer Weise sehr schnell lösen, können die erfindungsgemäßen Lyophilisate unmittelbar vor Anwendung rekonstituiert werden.

Im Zusammenhang mit der vorliegenden Erfindung wird unter einem Lyophilisat," ein Material verstanden, dass durch Trocknen im tiefgefrorenen Zustand im Hochvakuum durch Ausfrieren des Lösungsmittels, das im gefrorenen Zustand verdampft, erhalten wird. Ein auf diese Weise erhaltenes gefriergetrocknete Material ist sehr porös und behält sein ursprüngliches Volumen bei. Stoffwechsel-Funktionen, Enzym-Funktionen und/oder biologische Aktivität des Materials kommen nach Lyophilisierung zum Stillstand.

Im Zusammenhang mit der vorliegenden Erfindung wird unter einer "pharmazeutischen Zusammensetzung" oder einem "Arzneimittel" ein zu diagnostischen, therapeutischen und/oder prophylaktischen Zwecken verwendetes, also ein die Gesundheit eines menschlichen oder tierischen Körpers förderndes oder wiederherstellendes Gemisch verstanden, das mindestens einen natürlichen oder synthetisch hergestellten Wirkstoff umfasst, der die therapeutische Wirkung hervorruft.

Die pharmazeutische Zusammensetzung kann üblicherweise auf dem Fachgebiet verwendete Zusatzstoffe, beispielsweise Stabilisatoren, Fertigungsmittel, Trennmittel, Emulgatoren, Detergentien, Antioxidantien, kuchenbildende Mittel oder andere zur Herstellung pharmazeutischer Zusammensetzungen, insbesondere zur Herstellung flüssiger Darreichungsformen, verwendete Stoffe umfassen.

In bevorzugter Ausführungsform der Erfindung handelt es sich bei der erfindungsgemäß herzustellenden pharmazeutischen Zusammensetzung um eine flüssige pharmazeutische Zusammensetzung zur parenteralen Verabreichung.

Unter einer "parenteral" zu verabreichenden Darreichungsform oder pharmazeutischen Zusammensetzung" wird eine sterile pharmazeutische Zusammensetzung verstanden, die unter Umgehung des Magen-Darm-Traktes verabreicht wird. Die Vorteile der parenteralen Verabreichung, insbesondere gegenüber der oralen Verabreichung, bestehen vor allem darin, dass ein sehr schneller Wirkungseintritt möglich ist, dass Nebenwirkungen, wie zum Beispiel Erbrechen oder gastrointestinale Reizungen weitestgehend vermieden werden, dass Wirkstoffe gastrointestinal nicht inaktiviert werden, dass auch Wirkstoffe verabreicht werden können, die im Allgemeinen aus dem Magen-Darm-Kanal ungenügend resorbiert werden, dass der Blutspiegel des verabreichten Wirkstoffes vorausberechenbar ist und dass der sogenannte First-pass-Effekt vermieden wird.

Bei parenteral zu verabreichenden pharmazeutischen Zusammensetzungen handelt es sich insbesondere um Injektions- und Infusionslösungen. "Injektionen" oder "Injektionslösungen" sind Zubereitungen mit kleinen Volumina, insbesondere zwischen 1 und 20 ml, die als Lösung, Suspension oder Emulsion appliziert werden. Bei einer "Infusion" oder "Infusionslösung" werden Volumina, die größer als 100 ml sind, appliziert. Die häufigsten parenteralen Applikationswege sind die intravenöse (i.v.), die intramuskuläre (i.m.) und die subkutane (s.c.) Verabreichung. Die intravenöse Verabreichung ermöglicht die schnelle Zufuhr und Verabreichung von Wirkstoffen, die bei anderen parenteralen Verabreichungswegen gewebereizend wirken. Bei intramuskulären und subkutanen Injektionen muss die Isohydrie und Isotonie beachtet werden, da ansonsten lokale Unverträglichkeitserscheinungen auftreten können.

Erfindungsgemäß ist daher vorgesehen, dass die unter Verwendung des erfindungsgemäßen Lyophilisates herzustellende parenteral zu verabreichende pharmazeutische Zusammensetzung eine Injektionslösung oder Infusionslösung ist.

Erfindungsgemäß ist vorgesehen, dass Flupirtin in den erfindungsgemäßen Lyophilisaten entweder als Base oder als physiologisch verträgliches Salz vorliegen kann, wobei in bevorzugter Ausführungsform der Erfindung das erfindungsgemäße Lyophilisat mindestens 100 mg Flupirtin enthält, wobei sich diese Mengenangabe auf die Flupirtin-Base bezieht.

Unter "physiologisch verträglichen Salzen" von Flupirtin werden insbesondere solche Flupirtin-Salze verstanden, die als Säureadditionssalze vorliegen und die eine therapeutische Breite aufweisen, die durch einen ausreichend großen Abstand der Empfindlichkeitskurven der Flupirtin-Salze für deren therapeutische und letale Wirkung gekennzeichnet ist.

Beispiele geeigneter Säuren zur Herstellung physiologisch verträglicher Flupirtin-Salze umfassen Halogenwasserstoffsäuren, Schwefelsäure, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Bevorzugte Beispiele für geeignete Säuren sind Ameisen-, Essig-, Propion-, Bernstein-, Glykol-, Milch-, Äpfel-, Wein-, Citronen-, Ascorbin-, Malein-, Fumar-, Hydroxymalein-, Brenztrauben-, Phenylessig-, Benzoe-, p-Aminosalicyl-, Embon-, Methansulfon-, Ethansulfon-, Hydroxyethansulfon-, Ethylensulfon-, Halogenbenzolsulfon-, Toluolsulfon-, Naphthalinsulfonsäure, Sulfanilsäure und Salzsäure. Erfindungsgemäß besonders bevorzugt wird zur Herstellung des physiologisch verträglichen Flupirtin-Salzes Gluconsäure eingesetzt.

In bevorzugter Ausführungsform der Erfindung ist das physiologisch verträgliche Flupirtin-Salz daher das Formiat, Acetat, Propionat, Succinat, Glykolat, Lactat, Malat, Tartrat, Citrat, Maleat, Fumarat, Pyruvat, Phenylacetat, Benzoat, Embonat, Methansulfonat, Ethansulfonat, Hydroxyethansulfonat, Ethylensulfonat, Halogenbenzonsulfonat, Toluolsulfonat, Naphthalinsulfonat, Aminobenzolsulfonat oder Chlorid von Flupirtin. In besonders bevorzugter Ausführungsform der Erfindung ist das physiologisch verträgliche Flupirtin-Salz Flupirtingluconat.

Erfindungsgemäß ist ferner vorgesehen, dass das Flupirtin-Lyophilisat den Säure-Bestandteil des physiologisch verträglichen Flupirtin-Salzes in einer Menge von 60 mg bis 650 mg, vorzugsweise von 200 mg bis 400 mg, bezogen auf 100 mg Flupirtin, enthält.

In einer bevorzugten Ausführungsform der Erfindung enthält das erfindungsgemäße Flupirtin-Lyophilisat zusätzlich mindestens ein kuchenbildendes Mittel. Unter einem "kuchenbildenden Mittel" oder "Gerüstbildner" wird ein Mittel verstanden, das während und/oder nach Lyophilisierung eines Materials die Bildung eines porösen Kuchens mit sehr großer innerer Oberfläche unterstützt. In bevorzugter Ausführungsform enthält das erfindungsgemäße Flupirtin-Lyophilisat als kuchenbildendes Mittel Mannit, Saccharose oder Glycin. Erfindungsgemäß ist insbesondere vorgesehen, dass das kuchenbildende Mittel in dem erfindungsgemäßen Flupirtin-Lyophilisat in einer Menge von 10 mg bis 1000 mg, vorzugsweise von 30 mg bis 300 mg, bezogen auf 100 mg Flupirtin, enthalten ist.

In einer weiteren bevorzugten Ausführungsform der Erfindung ist vorgesehen, dass das erfindungsgemäße Flupirtin-Lyophilisat zusätzlich mindestens ein Antioxidans enthält. Unter "Antioxidantien" werden Hilfsstoffe verstanden, die die Oxidation einer Substanz, insbesondere eines Wirkstoffes hemmen, verzögern oder unterbinden können. Bei Antioxidantien kann es sich um Radikalfänger, leicht oxidierbare Stoffe oder Synergisten handelt. Bei der Oxidation organischer Verbindungen entstehen häufig radikalische Zwischenprodukte. Hilfsstoffe mit sterisch behinderten phenolischen Gruppen können leicht Wasserstoff-Radikale auf diese Zwischenprodukte übertragen, wobei sie selbst stabilere Moleküle bilden. Dadurch wird die oxidative Kettenreaktion unterbrochen. Radikalfänger werden insbesondere in nicht-wässrigen, lypophilen Systemen eingesetzt. In wässrigen Systemen werden hingegen vorrangig leicht oxidierbare Stoffe eingesetzt, wobei die Tatsache ausgenutzt wird, dass jeder zu schützende Stoff ein bestimmtes elektrisches Oxidationspotential besitzt. Das einzusetzendes Antioxidans weist dabei ein deutlich niedrigeres Oxidationspotential als die zu schützende Substanz auf. Bei Zutritt von Sauerstoff wird das Antioxidans dann leichter oxidiert als der zu schützende Stoff. Gleichzeitig wirkt der leicht oxidierbare Hilfsstoff als Protonen-Donator und dadurch stabilisierend. Pharmazeutisch nutzbare Stoffe dieser Art sind vor allem Ascorbinsäure mit einem Normaloxidationspotential von -0,04 V. Hydrogensulfite und Sulfite besitzen ein Normaloxidationspotential von +0,12 V. Bei Synergisten handelt es sich um eine Gruppe von Hilfsstoffen, die die Wirkung von Antioxidanzien entweder durch Regenerierung bereits oxidierter Hilfsstoff-Moleküle, durch Komplexierung von Schwermetallspuren, durch Zersetzung der Peroxide als Zwischenstufen der Oxidation oder durch Einstellen eines oxidationshemmenden pH-Wertes unterstützen.

In bevorzugter Ausführungsform der Erfindung enthält das erfindungsgemäße Lyophilisat als Antioxidans Natriumbisulfit oder Ascorbinsäure. Erfindungsgemäß ist vorgesehen, dass das Antioxidans im erfindungsgemäßen Flupirtin-Lyophilisat in einer Menge von 0,5 mg bis 10 mg, besonders bevorzugt in einer Menge von 2 mg bis 5 mg, bezogen auf 100 mg Flupirtin, enthalten ist.

In einer weiteren bevorzugten Ausführungsform der Erfindung ist vorgesehen, dass das erfindungsgemäße Flupiertin-Lyophilisat zusätzlich mindestens ein Detergens enthält. Im Zusammenhang mit der vorliegenden Erfindung wird unter einem "Detergens" eine organische grenzflächenaktive Substanz verstanden, die anionisch, kationisch, ampholytisch oder nichtionisch aufgebaut sein kann. Detergentien werden auch als Tenside bezeichnet. In der Pharmazie werden kationische Tenside hauptsächlich als Konservierungs- oder Desinfektionsmittel eingesetzt. Tenside können auch als W/O- oder O/W-Emulgatoren, Netzmittel, Lösungsvermittler, Schaumstabilisatoren oder Antischaummittel eingesetzt werden. Die Auswahl von Detergentien für bestimmte Aufgaben richtet sich sowohl nach der chemischen Konstitution der hydrophilen und lipophilen Gruppen der als Detergens eingesetzten Verbindung, da diese die Affinitäten zu den vorliegenden Phasen bestimmen, als auch nach den HLB-Werten, aber auch nach den Schmelz- beziehungsweise Erstarrungstemperaturen und nach den Viskositäten.

In bevorzugter Ausführungsform enthält das erfindungsgemäße Lyophilisat als Detergens ein Polyvinylpyrrolidon. Bei den Polyvinylpyrrolidonen handelt es sich um Polymerisationsprodukte des Vinylpyrrolidons. Handelsüblich ist eine Reihe von Fraktionen mit unterschiedlichen Molekül-Größen beziehungsweise Molekül-Kettenlängen. Eine hervorstechende Eigenschaft der Polyvinylpyrrolidone ist die gute Löslichkeit sowohl in Wasser als auch in polaren organischen Lösungsmitteln wie Alkoholen, Glykolen etc.. Erfindungsgemäß ist insbesondere vorgesehen, dass das Detergens, insbesondere Polyvinylpyrrolidon, im erfindungsgemäßen Flupirtin-Lyophilisat in einer Menge von 10 mg bis 150 mg, besonders bevorzugt in einer Menge von 10 mg bis 50 mg, bezogen auf 100 mg Flupirtin enthalten ist.

Die vorliegende Erfindung betrifft ebenfalls die Verwendung des erfindungsgemäßen Flupirtin-haltigen Lyophilisates zur Herstellung einer parenteral zu verabreichenden pharmazeutischen Zusammensetzung. Erfindungsgemäß ist dabei vorgesehen, dass das Lyophilisat zur Herstellung der parenteral zu verabreichenden pharmazeutischen Zusammensetzung verwendet wird, indem das Lyophilisat in einem wässrigen Medium und/oder einem organischen Lösungsmittel gelöst wird, wobei die parenteral zu verabreichende pharmazeutische Zusammensetzung erhalten wird. Erfindungsgemäß ist insbesondere vorgesehen, dass das Lyophilisat dabei bei Raumtemperatur gelöst wird. Als wässriges Medium wird erfindungsgemäß bevorzugt Wasser, besonders bevorzugt Wasser für Injektionszwecke verwendet. In einer anderen Ausführungsform der Erfindung ist vorgesehen, dass als wässriges Medium eine geeignete Pufferlösung verwendet wird. In einer weiteren Ausführungsform ist vorgesehen, dass das Lyophilisat zur Herstellung der parenteralen pharmazeutischen Zusammensetzung in einem Wasser-Lösungsmittel-Gemisch gelöst wird.

Die vorliegende Erfindung betrifft ebenfalls ein Verfahren zur Herstellung einer parenteral zu verabreichenden Flupirtin-haltigen pharmazeutischen Zusammensetzung, wobei ein erfindungsgemäße Flupirtin-haltiges Lyophilisat in einem wässrigen Medium und/oder einem organischen Lösungsmittel gelöst und eine gebrauchsfertige flüssige pharmazeutische Zusammensetzung erhalten wird.

Vorzugsweise das erfindungsgemäße Flupirtin-Lyophilisat bei Raumtemperatur gelöst wird. In besonders bevorzugter Ausführungsform wird das erfindungsgemäße Flupirtin-Lyophilisat in Wasser, insbesondere Wasser für Injektionszwecke, gelöst. Das Flupirtin-Lyophilisat kann aber auch in einer Pufferlösung oder in einem Wasser-Lösungsmittel-Gemisch gelöst werden.

Über das zum Auflösen verwendete Volumen des wässrigen Mediums kann die Isotonie der erhaltenen Lösung eingestellt werden. Vor der Applikation der herzustellenden parenteralen pharmazeutischen Zusammensetzung kann entschieden werden, wie das Lyophilisat zu rekonstituieren ist und ob gegebenenfalls eine Verdünnung erfolgen kann. So lässt sich aus dem erfindungsgemäßen Lyophilisat gleichermaßen eine intramuskuläre oder intravenöse Injektion herstellen. Die rekonstituierte wässrige Zubereitung kann auch als Zumischung zu gängigen Infusionslösungen verwendet werden.

Erfindungsgemäß ist daher vorgesehen, dass die unter Verwendung des erfindungsgemäßen Verfahrens hergestellte parenteral zu verabreichende pharmazeutische Zusammensetzung eine Injektionslösung ist. Wenn die herzustellende Injektionslösung intravenös appliziert werden soll, ist erfindungsgemäß vorgesehen, dass das erfindungsgemäße Lyophilisat, das vorzugsweise 100 mg Flupirtin enthält, in 3 bis 20 ml, vorzugsweise 9 bis 15 ml Wasser für Injektionszwecke, Pufferlösung oder Wasser/Lösungsmittel-Gemisch gelöst wird. Wenn die herzustellende Injektionslösung intramuskulär appliziert werden soll, ist erfindungsgemäß vorgesehen, dass das erfindungsgemäße Lyophilisat, das vorzugsweise 100 mg Flupirtin enthält, in 3 ml Wasser für Injektionszwecke, Pufferlösung oder Wasser/Lösungsmittel-Gemisch gelöst wird. In einer weiteren bevorzugten Ausführungsform der Erfindung ist vorgesehen, dass die parenteral zu verabreichende pharmazeutische Zusammensetzung eine Infusionslösung ist.

Die vorliegende Erfindung betrifft ebenfalls ein Verfahren zur Herstellung des erfindungsgemäßen Flupirtin-haltigen Lyophilisates, umfassend
a) Herstellung einer Flupirtin-Lösung durch Zugabe von Flupirtin-Base zu einem wässrigen Medium und Lösen darin, und
b) Gefriertrocknung der erhaltenen Flupirtin-Lösung.

Erfindungsgemäß ist also vorgesehen, dass im ersten Schritt zunächst Flupirtin in Basen-Form in einem wässrigen Medium gelöst wird. Soll das herzustellende Flupirtin-Lyophilisat ausschließlich Flupirtin-Base enthalten, wird die Flupirtin-Base vorzugsweise in Wasser, insbesondere Wasser für Injektionszwecke, gelöst. Soll das herzustellende Flupirtin-Lyophilisat ein physiologisch verträgliches Flupirtin-Salz enthalten, wird die Flupirtin-Base in einer wässrigen Lösung der entsprechenden Säure gelöst, wobei die Säure ausgewählt ist aus der Gruppe bestehend aus Glucon-, Ameisen-, Essig-, Propion-, Bernstein-, Glykol-, Milch-, Äpfel-, Wein-, Citronen-, Ascorbin-, Malein-, Fumar-, Hydroxymalein-, Brenztrauben-, Phenylessig-, Benzoe-, p-Aminosalicyl-, Embon-, Methansulfon-, Ethansulfon-, Hydroxyethansulfon-, Ethylensulfon-, Halogenbenzolsulfon-, Toluolsulfon-, Naphthalinsulfonsäure, Sulfanilsäure und Salzsäure. In bevorzugter Ausführungsform soll das herzustellende Lyophilisat Flupirtingluconat enthalten. Zur Herstellung eines Flupirtingluconat umfassenden Lyophilisats wird die Flupirtin-Base daher in einer Gluconsäure-Lösung gelöst.

In bevorzugter Ausführungsform der Erfindung wird das zum Lösen der Flupirtin-Base verwendete wässrige Medium, beispielsweise Wasser oder die Säure-Lösung, vor Zugabe der Flupirtin-Base auf eine über der Raumtemperatur liegende Temperatur erhitzt und bei dieser Temperatur gehalten. Erst nach Erwärmen wird Flupirtin dem erwärmten wässrigen Medium zugegeben und darin gelöst. In bevorzugter Ausführungsform wird das wässrige Medium auf eine Temperatur von 30°C bis 90°C, besonders bevorzugt 70°C erhitzt. Erfindungsgemäß ist ferner vorgesehen, dass Flupirtin unter Rühren in das vorzugsweise erwärmte wässrige Medium gegeben wird. Die Lösung wird dann solange gerührt, bis Flupirtin vollständig gelöst ist.

Erfindungsgemäß ist ferner vorgesehen, dass die so hergestellte Flupirtin-Lösung anschließend filtriert wird. Besonders bevorzugt wird dabei ein Filter mit einer Porenweite von 0,2 µm eingesetzt. Danach wird die vorzugsweise filtrierte Flupirtin enthaltene Lösung in Gefriertrocknungsflaschen eingefüllt, die dann mit Gefriertrockungsstopfen versehen werden. Zum Einfrieren der Flupirtin-Lösung werden die Gefriertrocknungsflaschen bei -45°C gelagert.

Erfindungsgemäß ist vorgesehen, dass die eigentliche Gefriertrocknung eine Haupttrocknung und eine Nachtrocknung umfasst. In bevorzugter Ausführungsform erfolgt die Haupttrocknung bei einer Temperatur von -37°C bis -23°C und einem Druck von 10 bis 100 mbar. In bevorzugter Ausführungsform erfolgt danach die Nachtrocknung bei einer Temperatur von 27°C und einem Druck von 0,0001 mbar. Nach der Gefriertrocknung wird der Gefriertrockner mit N₂ entspannt. Die das Flupirtin- Lyophilisat enthaltenden Flaschen werden dann vorzugsweise unter einer Stickstoffatmosphäre steril verschlossen.

Die vorliegende Erfindung betrifft ebenfalls die flüssige pharmazeutische Zusammensetzung zur parenteralen Verabreichung, die durch des erfindungsgemäßen Flupirtin-Lyophilisats erhältlich ist.

Die vorliegende Erfindung wird durch die nachfolgenden Beispiele näher erläutert.

### Beispiel 1: Herstellung eines Flupirtin-haltigen Lyophilisates

7,81 g Gluconsäure-d-lacton werden in 70 ml Wasser gelöst und dann auf 70°C erhitzt. Bei dieser Temperatur werden unter Rühren 3,33 g Flupirtin zugegeben und bis zur vollständigen Lösung gerührt. Das so erhaltene Gemisch wird durch einen Filter mit einer Porenweite von 0,2 µm filtriert. Nach der Filtration wird die Lösung in gefriergetrocknete Flaschen abgefüllt und mit geeigneten Gefriertrocknungsstopfen versehen. Zum Gefrieren des Füllgutes werden die Flaschen bei - 45°C gelagert. Die Haupttrocknung des Gefriertrocknungsprozesses erfolgt bei - 37°C bis -23°C und 100 bis 10 mbar. Die Nachtrocknung wird dann bei 27°C und 0,0001 mbar durchgeführt. Nach der Trocknung wird der Gefriertrockner mit N₂ entspannt. Die Flaschen werden dann unter Stickstoff-Atmosphäre verschlossen.

### Beispiel 2: Herstellung eines Flupirtin-haltigen Lyophilisates

7,81 g Gluconsäure-d-lacton werden in 70 ml Wasser gelöst und auf 70°C erhitzt. Bei dieser Temperatur werden unter Rühren 4,0 g Mannit und 3,33 g Flupirtin zugegeben und solange gerührt, bis die zugegebenen Verbindungen vollständig gelöst sind. Das so erhaltene Gemisch wird durch einen Filter mit einer Porenweite von 0,2 µm filtriert. Nach der Filtration wird die Lösung in Gefriertrocknungsflaschen abgefüllt und mit geeigneten Gefriertrocknungsstopfen versehen. Zum Gefrieren des Füllgutes werden die Flaschen bei -45°C gelagert. Die Haupttrocknung des Gefriertrocknungsprozesses erfolgt bei -37°C bis -23°C und 100 bis 10 mbar. Die Nachtrocknung wird bei 27°C und 0,0001 mbar durchgeführt. Nach der Trocknung wird der Gefriertrockner mit N₂ entspannt. Danach werden die Flaschen unter Stickstoff-Atmosphäre verschlossen.

### Beispiel 3: Herstellung eines Flupirtin-haltigen Lyophilisates

7,81 g Gluconsäure-d-lacton werden in 70 ml Wasser gelöst und auf 70°C erhitzt. Bei dieser Temperatur werden unter Rühren 7,5 g Saccharose, 0,4 g Polyvinylpyrrolidon (MG etwa 11500; Kollidon PF17; BASF) und 3,33 g Flupirtin zugegeben. Die Lösung wird solange gerührt, bis die zugegebenen Substanzen vollständig gelöst sind. Das so erhaltene Gemisch wird durch einen Filter mit einer Porenweite von 0,2 µm filtriert. Nach der Filtration wird die Lösung in Gefriertrocknungsflaschen abgefüllt und mit geeigneten Gefriertrocknungsstopfen versehen. Zum Gefrieren des Füllgutes werden die Flaschen bei -45°C gelagert. Die Haupttrocknung des Gefriertrocknungsprozesses erfolgt bei -37°C bis -23°C und 100 bis 10 mbar. Die Nachtrocknung wird bei 27°C und 0,0001 mbar durchgeführt. Nach der Trocknung wird der Gefriertrockner mit N₂ entspannt. Die Flaschen werden dann unter Stickstoffatmosphäre verschlossen.

### Beispiel 4: Zusammensetzung eines Flupirtin-Lyophilisates

Eine Flasche enthält:
100,00 mg Flupirtin
257,85 mg Gluconsäure
300,00 mg Mannit.

### Beispiel 5: Zusammensetzung eines Flupirtin-Lyophilisates

Eine Flasche enthält:
100,00 mg Flupirtin
257,85 mg Gluconsäure
225,00 mg Saccharose
12,00 mg Polyvinylpyrrolidon (Kollidon 17 PF, BASF).

### Beispiel 6: Zusammensetzung eines Flupirtin-Lyophilisates

Eine Flasche enthält:
100,00 mg Flupirtin
257,85 mg Gluconsäure
120,00 mg Mannit
4,50 mg Natriumbisulfit

### Beispiel 7: Zusammensetzung eines Flupirtin-Lyophilisates

Eine Flasche enthält:
100,00 mg Flupirtin
257,85 mg Gluconsäure
207,00 mg Mannit

Beispiel 8: **Herstellung einer flüssigen pharmazeutischen Zusammensetzung**

Eine Flasche des in Beispiel 7 beschriebenen Lyophilisats wird mit 3 ml Wasser für Injektionszwecke versetzt. Nach gelegentlichem Umschwenken wird nach etwa 1 min eine klare Lösung erhalten. Die Lösung ist mit 980 mosmol/kg deutlich hypertonisch.

### Beispiel 9: Herstellung einer flüssigen pharmazeutischen Zusammensetzung

Eine Flasche des Lyophilisats aus Beispiel 7 wird mit 9 ml Wasser für Injektionszwecke versetzt. Unter gelegentlichem Rühren wird nach etwa 1 Minute eine klare Lösung erhalten. Die Lösung ist mit 305 mosmol/kg nahezu isotonisch.

## Patentansprüche

1. Lyophilisat, das den Wirkstoff Flupirtin in Basen-Form oder als physiologisch verträgliches Salz enthält und zur Herstellung einer parenteral zu verabreichenden pharmazeutischen Zusammensetzung eingesetzt werden kann.

2. Lyophilisat nach Anspruch 1, enthaltend mindestens 100 mg Flupirtin.

3. Lyophilisat nach Anspruch 1, wobei das physiologisch verträgliche Salz ein Säureadditionssalz von Flupirtin ist.

4. Lyophilisat nach Anspruch 3, wobei der Säure-Bestandteil des Salzes ausgewählt ist aus der Gruppe bestehend aus Glucon-, Ameisen-, Essig-, Propion-, Bernstein-, Glykol-, Milch-, Äpfel-, Wein-, Citronen-, Ascorbin-, Malein-, Fumar-, Hydroxymalein-, Brenztrauben-, Phenylessig-, Benzoe-, p-Aminosalicyl-, Embon-, Methansulfon-, Ethansulfon-, Hydroxyethansulfon-, Ethylensulfon-, Halogenbenzolsulfon-, Toluolsulfon-, Naphthalinsulfon-, Sulfanil- und Salzsäure.

5. Lyophilisat nach Anspruch 3 oder 4, wobei der Säure-Bestandteil des Salzes in einer Menge von 60 mg bis 650 mg, bezogen auf 100 mg Flupirtin, enthalten ist.

6. Lyophilisat nach Anspruch 5, wobei der Säure-Bestandteil in einer Menge von 200 mg bis 400 mg, bezogen auf 100 mg Flupirtin, enthalten ist.

7. Lyophilisat nach einem der Ansprüche 1 bis 6, zusätzlich enthaltend mindestens ein kuchenbildendes Mittel.

8. Lyophilisat nach Anspruch 7, wobei das kuchenbildende Mittel Mannit, Saccharose oder Glycin ist.

9. Lyophilisat nach Anspruch 7 oder 8, wobei das kuchenbildende Mittel in einer Menge von 10 mg bis 1000 mg, bezogen auf 100 mg Flupirtin, enthalten ist.

10. Lyophilisat nach Anspruch 9, wobei das kuchenbildende Mittel in einer Menge von 30 mg bis 300 mg, bezogen auf 100 mg Flupirtin, enthalten ist.

11. Lyophilisat nach einem der Ansprüche 1 bis 10, zusätzlich enthaltend mindestens ein Antioxidans.

12. Lyophilisat nach Anspruch 11, wobei das Antioxidans Natriumbisulfit oder Ascorbinsäure ist.

13. Lyophilisat nach Anspruch 11 oder 12, wobei das Antioxidans in einer Menge von 0,5 mg bis 10 mg, bezogen auf 100 mg Flupirtin, enthalten ist.

14. Lyophilisat nach Anspruch 13, wobei das Antioxidans in einer Menge von 2 mg bis 5 mg, bezogen auf 100 mg Flupirtin, enthalten ist.

15. Lyophilisat nach einem der Ansprüche 1 bis 14, zusätzlich enthaltend ein Detergens.

16. Lyophilisat nach Anspruch 15, wobei das Detergens ein Polyvinylpyrrolidon ist.

17. Lyophilisat nach Anspruch 15 oder 16, wobei das Detergens in einer Menge von 10 mg bis 150 mg, bezogen auf 100 mg Flupirtin, enthalten ist.

18. Lyophilisat nach Anspruch 17, wobei das Detergens in einer Menge von 10 mg bis 50 mg, bezogen auf 100 mg Flupirtin, enthalten ist.

19. Lyophilisat nach einem der Ansprüche 1 bis 18, wobei die parenteral zu verabreichende pharmazeutische Zusammensetzung eine Injektionslösung oder Infusionslösung ist.

20. Verwendung eines Flupirtin-Lyophilisats nach einem der Ansprüche 1 bis 19 zur Herstellung einer parenteral zu verabreichenden pharmazeutischen Zusammensetzung.

21. Verwendung nach Anspruch 20, wobei das Lyophilisat in einem wässrigen Medium und/oder einem organischen Lösungsmittel gelöst und die parenteral zu verabreichende pharmazeutische Zusammensetzung erhalten wird.

22. Verwendung nach Anspruch 21, wobei das Lyophilisat in Wasser für Injektionszwecke gelöst wird.

23. Verwendung nach Anspruch 21, wobei das Lyophilisat in einer Pufferlösung gelöst wird.

24. Verwendung nach Anspruch 21, wobei das Lyophilisat in einem Wasser/Lösungsmittel-Gemisch gelöst wird.

25. Verwendung nach einem der Ansprüche 21 bis 24, wobei das Lyophilisat bei Raumtemperatur gelöst wird.

26. Verfahren zur Herstellung einer parenteral zu verabreichenden Flupirtin-haltigen pharmazeutischen Zusammensetzung, wobei ein Flupirtin-haltiges Lyophilisat nach einem der Ansprüche 1 bis 19 in einem wässrigen Medium und/oder einem organischen Lösungsmittel gelöst und eine gebrauchsfertige flüssige pharmazeutische Zusammensetzung erhalten wird.

27. Verfahren nach Anspruch 26, wobei das Lyophilisat in Wasser für Injektionszwecke gelöst wird.

28. Verfahren nach Anspruch 26, wobei das Lyophilisat in einer Pufferlösung gelöst wird.

29. Verfahren nach Anspruch 26, wobei das Lyophilisat in einem Wasser/Lösungsmittel-Gemisch gelöst wird.

30. Verfahren nach einem der Ansprüche 26 bis 29, wobei das Lyophilisat bei Raumtemperatur gelöst wird.

31. Verfahren nach einem der Ansprüche 26 bis 30, wobei die parenteral zu verabreichende pharmazeutische Zusammensetzung eine Injektionslösung ist.

32. Verfahren nach Anspruch 31, wobei die Injektionslösung intravenös applizierbar ist.

33. Verfahren nach Anspruch 32, wobei das Lyophilisat zur Herstellung der intravenös applizierbaren Injektionslösung in 3 bis 20 ml, vorzugsweise 9 bis 15 ml Wasser für Injektionszwecke gelöst wird.

34. Verfahren nach Anspruch 31, wobei die Injektionslösung intramuskulär applizierbar ist.

35. Verfahren nach Anspruch 34, wobei das Lyophilisat zur Herstellung der intramuskulär applizierbaren Injektionslösung in 3 ml Wasser für Injektionszwecke gelöst wird.

36. Verfahren nach einem der Ansprüche 26 bis 30, wobei die parenteral zu verabreichende pharmazeutische Zusammensetzung eine Infusionslösung ist.

37. Verfahren zur Herstellung eines Flupirtin-haltigen Lyophilisats nach einem der Ansprüche 1 bis 19, umfassend
a) Herstellung einer Flupirtin-Lösung durch Zugabe der Flupirtin-Base zu einem wässrigen Medium und Lösen darin, und
b) Gefriertrocknung der erhaltenen Flupirtin-Lösung.

38. Verfahren nach Anspruch 37, wobei die Flupirtin-Lösung in Wasser hergestellt wird.

39. Verfahren nach Anspruch 37, wobei die Flupirtin-Lösung in einer wässrigen Säure-Lösung hergestellt wird.

40. Verfahren nach Anspruch 39, wobei die Säure-Lösung durch Lösen einer Säure ausgewählt aus der Gruppe bestehend aus Glucon-, Ameisen-, Essig-, Propion-, Bernstein-, Glykol-, Milch-, Äpfel-, Wein-, Citronen-, Ascorbin-, Malein-, Fumar-, Hydroxymalein-, Brenztrauben-, Phenylessig-, Benzoe-, p-Aminosalicyl-, Embon-, Methansulfon-, Ethansulfon-, Hydroxyethansulfon-, Ethylensulfon-, Halogenbenzolsulfon-, Toluolsulfon-, Naphthalinsulfon-, Sulfanil- und Salzsäure in Wasser hergestellt wird.

41. Verfahren nach einem der Ansprüche 38 bis 40, wobei das zum Lösen der Flupirtin-Base verwendete wässrige Medium auf eine über Raumtemperatur liegende Temperatur erhitzt und dann die Flupirtin-Base zugegeben wird.

42. Verfahren nach Anspruch 41, wobei das wässrige Medium auf 30°C bis 90°C erhitzt wird.

43. Verfahren nach Anspruch 42, wobei das wässrige Medium auf 70°C erhitzt wird.

44. Verfahren nach einem der Ansprüche 37 bis 43, wobei die Flupirtin-Base unter Rühren in das, vorzugsweise erhitzte wässrige Medium gegeben und unter Rühren darin gelöst wird.

45. Verfahren nach einem der Ansprüche 37 bis 44, wobei die hergestellte Flupirtin-Lösung vor Gefriertrocknung filtriert wird.

46. Verfahren nach Anspruch 45, wobei zur Filtration ein Filter mit einer Porenweite von 0,2 µm eingesetzt wird.

47. Verfahren nach einem der Ansprüche 37 bis 46, wobei die Flupirtin-Lösung nach Filtration in Gefriertrocknungsflaschen abgefüllt wird und diese dann mit Gefriertrocknungsstopfen versehen werden.

48. Verfahren nach einem der Ansprüche 37 bis 47, wobei die Flupirtin-Lösung bei -45°C gelagert wird.

49. Verfahren nach einem der Ansprüche 37 bis 48, wobei die Gefriertrocknung eine Haupttrocknung und eine Nachtrocknung umfasst.

50. Verfahren nach Anspruch 49, wobei die Haupttrocknung bei einer Temperatur von -37°C bis -23°C und einem Druck von 10 bis 100 mbar erfolgt.

51. Verfahren nach Anspruch 49 oder 50, wobei die Nachtrocknung bei einer Temperatur von 27°C und einem Druck von 0,0001 mbar erfolgt.

52. Verfahren nach einem der Ansprüche 37 bis 51, wobei nach der Gefriertrocknung die das Lyophilisat enthaltenden Flaschen unter einer Stickstoff-Atmosphäre verschlossen werden.

53. Flüssige Flupirtin-haltige pharmazeutische Zusammensetzung zur parenteralen Verabreichung, herstellbar durch Lösen eines Flupirtin-haltigen Lyophilisats nach einem der Ansprüche 1 bis 19.

## Claims

1. Lyophilisate containing the agent flupirtine in the form of a base or as physiologically compatible salt, and being suitable for the preparation of a pharmaceutical composition for parenteral administration.

2. Lyophilisate according to claim 1 containing at least 100 mg of flupirtine.

3. Lyophilisate according to claim 1, wherein the physiologically compatible salt is an acid addition salt of flupirtine.

4. Lyophilisate according to claim 3, wherein the acidic component of the salt is selected from the group consisting of gluconic, formic, acetic, propionic, succinic, glycolic, lactic, malic, tartaric, citric, ascorbic, maleic, fumaric, hydroxymaleic, pyruvic, phenylacetic, benzoic, p-aminosalicylic, embonic, methanesulfonic, ethanesulfonic, hydroxyethanesulfonic, ethylenesulfonic, halogen benzene sulfonic, toluene sulfonic, naphthalene sulfonic, sulfanilic and hydrochloric acid.

5. Lyophilisate according to claim 3 or 4, wherein the acidic component of the salt is contained in an amount of 60 mg to 650 mg based on 100 mg of flupirtine.

6. Lyophilisate according to claim 5, wherein the acidic component is contained in an amount of 200 mg to 400 mg based on 100 mg of flupirtine.

7. Lyophilisate according to any of claims 1 to 6 additionally containing at least one cake forming agent.

8. Lyophilisate according to claim 7, wherein the cake forming agent is mannitol, sucrose or glycine.

9. Lyophilisate according to claim 7 or 8, wherein the cake forming agent is contained in an amount of 10 mg to 1000 mg based on 100 mg of flupirtine.

10. Lyophilisate according to claim 9, wherein the cake forming agent is contained in an amount of 30 mg to 300 mg based on 100 mg of flupirtine.

11. Lyophilisate according to any one of claims 1 to 10, additionally containing at least one antioxidant.

12. Lyophilisate according to claim 11, wherein the antioxidant is sodium bisulfite or ascorbic acid.

13. Lyophilisate according to claim 11 or 12, wherein the antioxidant is contained in an amount of 0.5 mg to 10 mg based on 100 mg of flupirtine.

14. Lyophilisate according to claim 13, wherein the antioxidant is contained in an amount of 2 mg to 5 mg based on 100 mg of flupirtine.

15. Lyophilisate according to any one of claims 1 to 14, additionally containing at least one detergent.

16. Lyophilisate according to claim 15, wherein the detergent is a polyvinylpyrrolidone.

17. Lyophilisate according to claim 15 or 16, wherein the detergent is contained in an amount of 10 mg to 150 mg based on 100 mg of flupirtine.

18. Lyophilisate according to claim 17, wherein the detergent is contained in an amount of 10 mg to 50 mg based on 100 mg of flupirtine.

19. Lyophilisate according to any of claims 1 to 18, wherein the pharmaceutical composition to be administered parenterally is a solution for injection or a solution for infusion.

20. Use of a flupirtine lyophilisate according to any one of claims 1 to 19 for the preparation of a pharmaceutical composition for parenteral administration.

21. Use according to claim 20, wherein the lyophilisate is dissolved in an aqueous medium and/or an organic solvent, and the pharmaceutical composition for parenteral administration is obtained.

22. Use according to claim 21, wherein the lyophilisate is dissolved in water for injection purposes.

23. Use according to claim 21, wherein the lyophilisate is dissolved in a buffer solution.

24. Use according to claim 21, wherein the lyophilisate is dissolved in a mixture of water and solvent.

25. Use according to any of claims 21 to 24, wherein the lyophilisate is dissolved at room temperature.

26. Method for the preparation of a flupirtine-containing pharmaceutical composition for parenteral administration, wherein a flupirtine-containing lyophilisate according to any one of claims 1 to 19 is dissolved in an aqueous medium and/or an organic solvent, and wherein a ready-made liquid pharmaceutical composition is obtained.

27. Method according to claim 26, wherein the lyophilisate is dissolved in water for injection purposes.

28. Method according to claim 26, wherein the lyophilisate is dissolved in a buffer solution.

29. Method according to claim 26, wherein the lyophilisate is dissolved in a mixture of water and solvent.

30. Method according to any of claims 26 to 29, wherein the lyophilisate is dissolved at room temperature.

31. Method according to any of claims 26 to 30, wherein the pharmaceutical composition to be administered parenterally is a solution for injection.

32. Method according to claim 31, wherein the solution for injection is administrable intravenously.

33. Method according to claim 32, wherein the lyophilisate for the preparation of an injection solution which is administrable intravenously is dissolved in 3 to 20 ml, preferably 9 to 15 ml, water for injection purposes.

34. Method according to claim 31, wherein the solution for injection is administrable intramuscularly.

35. Method according to claim 34, wherein the lyophilisate for the preparation of said injection solution for intramuscular administration is dissolved in 3 ml of water for injection purposes.

36. Method according to any of claims 26 to 30, wherein the pharmaceutical composition for parenteral administration is an infusion solution.

37. Method for the preparation of a flupirtine-containing lyophilisate according to any of claims 1 to 19, comprising
a) the preparation of a flupirtine solution by adding the flupirtine base to an aqueous medium and dissolving it therein, and
b) freeze-drying the obtained flupirtine solution.

38. Method according to claim 37, wherein the flupirtine solution is prepared in water.

39. Method according to claim 37, wherein the flupirtine solution is prepared in an aqueous acid solution.

40. Method according to claim 39, wherein the acid solution is prepared by dissolving in water an acid selected from the group consisting of gluconic, formic, acetic, propionic, succinic, glycolic, lactic, malic, tartaric, citric, ascorbic, maleic, fumaric, hydroxymaleic, pyruvic, phenylacetic, benzoic, p-aminosalicylic, embonic, methanesulfonic, ethanesulfonic, hydroxyethanesulfonic, ethylenesulfonic, halogen benzene sulfonic, toluene sulfonic, naphthalene sulfonic, sulfanilic and hydrochloricd acid.

41. Method according to any one of claims 38 to 40, wherein the aqueous medium used for dissolving the flupirtine base is heated to a temperature above room temperature followed by adding the flupirtine base.

42. Method according to claim 41, wherein the aqueous medium is heated to a temperature of from 30 °C to 90 °C.

43. Method according to claim 42, wherein the aqueous medium is heated to 70°C.

44. Method according to any of claims 37 to 43, wherein the flupirtine base is added with stirring to the preferably heated aqueous medium and dissolved therein with stirring.

45. Method according to any of claims 37 to 44, wherein the prepared flupirtine solution is filtered before freeze-drying.

46. Method according to claim 45, wherein a filter having a pore size of 0.2 µm is used for filtration.

47. Method according to any of claims 37 to 46, wherein, after filtration, the flupirtine solution is filled into lyophilisation flasks which are then sealed with lyophilisation plugs.

48. Method according to any of claims 37 to 47, wherein the flupirtine solution is stored at -45 °C.

49. Method according to any of claims 37 to 48, wherein the freeze-drying comprises a main drying and a secondary drying.

50. Method according to claim 49, wherein the main drying takes place at a temperature of -37 °C to -23 °C, and at a pressure of 10 to 100 mbar.

51. Method according to claim 49 or 50, wherein the secondary drying takes place at a temperature of 27 °C and at a pressure of 0.0001 mbar.

52. Method according to any of claims 37 to 51, wherein after freeze-drying the lyophilisate-containing flasks are sealed under nitrogen atmosphere.

53. Liquid flupirtine-containing pharmaceutical composition for parenteral administration which is preparable by dissolving a flupirtine-containing lyophilisate according to any of claims 1 to 19.

## Revendications

1. Lyophilisat qui contient le principe actif flupirtine sous sa forme base ou sous forme d'un sel compatible d'un point de vue physiologique, et qui peut être utilisé pour préparer une composition pharmaceutique pour administration parentérale.

2. Lyophilisat selon la revendication 1, contenant au moins 100 mg de flupirtine.

3. Lyophilisat selon la revendication 1, dans lequel le sel compatible d'un point de vue physiologique est un sel d'addition avec un acide de la flupirtine.

4. Lyophilisat selon la revendication 3, dans lequel le constituant acide du sel est choisi dans le groupe consistant en l'acide gluconique, l'acide formique, l'acide acétique, l'acide propionique, l'acide succinique, l'acide glycolique, l'acide lactique, l'acide malique, l'acide tartrique, l'acide citrique, l'acide ascorbique, l'acide maléique, l'acide fumarique, l'acide hydroxymaléique, l'acide pyruvique, l'acide phénylacétique, l'acide benzoïque, l'acide p-aminosalicylique, l'acide embonique, l'acide méthanesulfonique, l'acide éthanesulfonique, l'acide hydroxyéthanesulfonique, l'acide éthylène-sulfonique, les acides halogénobenzènesulfoniques, l'acide toluènesulfonique, l'acide naphtalènesulfonique, l'acide sulfanilique et l'acide chlorhydrique.

5. Lyophilisat selon la revendication 3 ou 4, dans lequel le constituant acide du sel est présent en une quantité de 60 mg à 650 mg pour 100 mg de flupirtine.

6. Lyophilisat selon la revendication 5, dans lequel le constituant acide est présent en une quantité de 200 mg à 400 mg pour 100 mg de flupirtine.

7. Lyophilisat selon l'une des revendications 1 à 6, contenant en outre au moins un agent de formation d'un gâteau.

8. Lyophilisat selon la revendication 7, dans lequel l'agent de formation d'un gâteau est le mannitol, le saccharose ou la glycine.

9. Lyophilisat selon la revendication 7 ou 8, dans lequel l'agent de formation d'un gâteau est présent en une quantité de 10 mg à 1000 mg pour 100 mg de flupirtine.

10. Lyophilisat selon la revendication 9, dans lequel l'agent de formation d'un gâteau est présent en une quantité de 30 mg à 300 mg pour 100 mg de flupirtine.

11. Lyophilisat selon l'une des revendications 1 à 10, contenant en outre au moins un antioxydant.

12. Lyophilisat selon la revendication 11, dans lequel l'antioxydant est le bisulfite de sodium ou l'acide ascorbique.

13. Lyophilisat selon la revendication 11 ou 12, dans lequel l'antioxydant est présent en une quantité de 0,5 mg à 10 mg pour 100 mg de flupirtine.

14. Lyophilisat selon la revendication 13, dans lequel l'antioxydant est présent en une quantité de 2 mg à 5 mg pour 100 mg de flupirtine.

15. Lyophilisat selon l'une des revendications 1 à 14, contenant en outre un détergent.

16. Lyophilisat selon la revendication 15, dans lequel le détergent est une polyvinylpyrrolidone.

17. Lyophilisat selon la revendication 15 ou 16, dans lequel le détergent est présent en une quantité de 10 mg à 150 mg pour 100 mg de flupirtine.

18. Lyophilisat selon la revendication 17, dans lequel le détergent est présent en une quantité de 10 mg à 50 mg pour 100 mg de flupirtine.

19. Lyophilisat selon l'une des revendications 1 à 18, pour lequel la composition pharmaceutique pour administration parentérale est une solution injectable ou une solution pour perfusion.

20. Utilisation d'un lyophilisat de flupirtine selon l'une des revendications 1 à 19 pour préparer une composition pharmaceutique pour administration parentérale.

21. Utilisation selon la revendication 20, pour laquelle le lyophilisat est soumis à une dissolution dans un milieu aqueux et/ou dans un solvant organique, et on obtient la composition pharmaceutique pour administration parentérale.

22. Utilisation selon la revendication 21, pour laquelle le lyophilisat est soumis à une dissolution dans de l'eau pour injection.

23. Utilisation selon la revendication 21, pour laquelle le lyophilisat est soumis à une dissolution dans une solution tampon.

24. Utilisation selon la revendication 21, pour laquelle le lyophilisat est soumis à une dissolution dans un mélange eau/solvant.

25. Utilisation selon l'une des revendications 21 à 24, pour laquelle le lyophilisat est soumis à une dissolution à la température ambiante.

26. Procédé de préparation d'une composition pharmaceutique contenant de flupirtine pour administration parentérale, dans lequel on dissout un lyophilisat contenant de la flupirtine selon l'une des revendications 1 à 19 dans un milieu aqueux et/ou dans un solvant organique, et on obtient une composition pharmaceutique liquide prête à l'emploi.

27. Procédé selon la revendication 26, dans lequel le lyophilisat est soumis à une dissolution dans de l'eau pour injection.

28. Procédé selon la revendication 26, dans lequel le lyophilisat est soumis à une dissolution dans une solution tampon.

29. Procédé selon la revendication 26, dans lequel le lyophilisat est soumis à une dissolution dans un mélange eau/solvant.

30. Procédé selon l'une des revendications 26 à 29, dans lequel le lyophilisat est soumis à une dissolution à la température ambiante.

31. Procédé selon l'une des revendications 26 à 30, dans lequel la composition pharmaceutique pour administration parentérale est une solution injectable.

32. Procédé selon la revendication 31, dans lequel la solution injectable peut être administrée par voie intraveineuse.

33. Procédé selon la revendication 32, dans lequel le lyophilisat est, pour la préparation de la solution injectable pouvant être administrée par voie intraveineuse, soumis à une dissolution dans 3 à 20 ml et de préférence dans 9 à 15 ml d'eau pour injection.

34. Procédé selon la revendication 31, dans lequel la solution injectable peut être administrée par voie intramusculaire.

35. Procédé selon la revendication 34, dans lequel le lyophilisat est, pour la préparation de la solution injectable pouvant être administrée par voie intramusculaire, soumis à une dissolution dans 3 ml d'eau pour injection.

36. Procédé selon l'une des revendications 26 à 30, dans lequel la composition pharmaceutique pour administration parentérale est une solution pour perfusion.

37. Procédé de préparation d'un lyophilisat contenant de la flupirtine selon l'une des revendications 1 à 19, comprenant
a) la préparation d'une solution de flupirtine par addition de la flupirtine base à un milieu aqueux, ou par dissolution dans ce dernier, et
b) lyophilisation de la solution de flupirtine obtenue.

38. Procédé selon la revendication 37, dans lequel la solution de flupirtine est préparée dans de l'eau.

39. Procédé selon la revendication 37, dans lequel la solution de flupirtine est préparée dans une solution aqueuse d'un acide.

40. Procédé selon la revendication 39, dans lequel la solution d'un acide est préparée par dissolution, dans de l'eau, d'un acide choisi dans le groupe consistant en l'acide gluconique, l'acide formique, l'acide acétique, l'acide propionique, l'acide succinique, l'acide glycolique, l'acide lactique, l'acide malique, l'acide tartrique, l'acide citrique, l'acide ascorbique, l'acide maléique, l'acide fumarique, l'acide hydroxymaléique, l'acide pyruvique, l'acide phénylacétique, l'acide benzoïque, l'acide p-aminosalicylique, l'acide embonique, l'acide méthanesulfonique, l'acide éthanesulfonique, l'acide hydroxyéthanesulfonique, l'acide éthylène-sulfonique, les acides halogénobenzènesulfoniques, l'acide toluènesulfonique, l'acide naphtalènesulfonique, l'acide sulfanilique et l'acide chlorhydrique.

41. Procédé selon l'une des revendications 38 à 40, dans lequel le milieu aqueux utilisé pour dissoudre la flupirtine base est chauffé à une température supérieure à la température ambiante, puis on ajoute la flupirtine base.

42. Procédé selon la revendication 41, dans lequel le milieu aqueux est chauffé à une température de 30 à 90°C.

43. Procédé selon la revendication 42, dans lequel le milieu aqueux est chauffé à 70°C.

44. Procédé selon l'une des revendications 37 à 43, dans lequel la flupirtine base est, sous agitation, ajoutée au milieu aqueux de préférence chauffé, et y est soumise à une dissolution sous agitation.

45. Procédé selon l'une des revendications 37 à 44, dans lequel la solution de flupirtine ainsi préparée est filtrée avant lyophilisation.

46. Procédé selon la revendication 45, dans lequel on utilise pour la filtration un filtre ayant un diamètre de pore de 0,2 µm.

47. Procédé selon l'une des revendications 37 à 46, dans lequel la solution de flupirtine est, après filtration, transvasée dans des flacons de lyophilisation, ces derniers étant ensuite pourvus de bouchons de lyophilisation.

48. Procédé selon i'-une des revendications 37 à 47, dans lequel la solution de flupirtine est stockée à -45°C.

49. Procédé selon l'une des revendications 37 à 48, dans lequel la lyophilisation comprend une dessiccation principale et une dessiccation subséquente.

50. Procédé selon la revendication 49, dans lequel la dessiccation principale a lieu à une température de -37 à -23°C et sous une pression de 10 à 100 mbar.

51. Procédé selon la revendication 49 ou 50, dans lequel la dessiccation subséquente est réalisée à une température de 27°C et sous une pression de 0,0001 mbar.

52. Procédé selon l'une des revendications 37 à 51, dans lequel, après la lyophilisation, les flacons contenant le lyophilisat sont obturés sous atmosphère d'azote.

53. Composition pharmaceutique liquide contenant de la flupirtine pour administration parentérale, pouvant être préparée par dissolution d'un lyophilisat contenant de la flupirtine selon l'une des revendications 1 à 19.
